# EUROPEAN PATENT SPECIFICATION

(11) **EP 0 913 838 B1**
(45) Date of publication and mention of the grant of the patent: **05.06.2002**
(21) Application number: 98117817.1
(22) Date of filing: 19.09.1998
(51) Int. Cl.: G21K 1/00, A61B 6/06

(54) **Apparatus for radiological examinations**
Röntgenuntersuchungsgerät
Dispositif d'examen radiologique

(30) Priority: 28.10.1997 IT PN970059
(43) Date of publication of application: 06.05.1999
(73) Proprietor: Medys S.r.l., 10024 Moncalieri Torino (IT)
(72) Inventor: Pagano, Gaetano, 50142 Firenze (IT)
(74) Representative: Giugni, Valter

(56) References cited:
- EP-A- 0 402 082
- WO-A-96/18282
- GB-A- 2 024 562
- US-A- 4 731 806
- US-A- 4 982 419

## Description

The present invention refers to apparatuses for radiological examinations, and in particular the device thereof which is used to hold the photographic film or plate onto which the image of the part of the body of the patient undergoing examination is impressed.

Radiology is commonly known to be the medical science concerned with the use of ionizing radiations, such as in particular x-rays, to purposes of diagnosis and treatment of disease, wherein a part of the body of the patient is substantially exposed to a beam of radiations that move therethrough in a selective manner and finally impress the photographic film, thereby delivering an image from which a number of valuable clinical information can be derived concerning the examined part of the body.

A major problem encountered in connection with radiological examinations is represented by radiations scattering in the body of the patient so as to give rise to interferences on the image generated by direct radiations.

As a result, radiological apparatuses require being provided with an arrangement that enables them to reduce the effects of such scattered radiations. Such an arrangement is known in the art by the name of Potter-Bucky grid or even Bucky diaphragm. This is practically a grid formed by an assembly of lead strips resembling an open venetian blind, which is placed between the body of the patient being x-rayed and the photographic film or plate to be impressed.

This grid has the task of filtering out the rays being scattered by the irradiated elements , which would impair the quality of the impressed image.

Said rectilinear lead strips of the grid are usually spaced from each other through the interposition of strips of a x-ray transparent material (which was usually wood in the past, now commonly replaced with plastics or aluminium) therebetween. The strips are oriented so as to cause all of them to be directed towards a virtual line, ie. the focal line. The focus of the x-ray emitting tube is placed in this focal line. The primary rays, after having moved through the body of the patient, come up against the grid, the strips of which oppose them just a minimum of absorption surface. Conversely, the oblique rays emitted by scattering by the body of the patient are hindered from passing therethrough by the strips of the grid. In this way, the grid therefore carries out a selective absorption of scattered radiations.

The first moving grids have been provided by assembling the strips thereof according to a cylindrical surface, whose axis coincided with the focal line. During the blanking movement, the grid remained focused. However, this particular embodiment had a major drawback in that the examination tables were provided with a cylindrical surface with the grid placed thereunder or, better, if the surface was plane, the need arised to place the grid thereabove, with appropriate devices on all sides, for the convenience of the patient.

The current use of plane grids has actually represented a major progress in this sector. However, these grids still have a drawback in that the focusing of the radiation is not kept all over the grid during the movement. Let's now indicate with R the ratio of the height h to the distance d of the lead strips: R = h/d.

A grid having a high ratio R requires the central ray to be focused in an extremely accurate manner. A centering or focusing error would in fact entail a considerable absorption effect. For example, a 2° focusing error on a grid having a R ratio of 6.5 would entail a 64% transmission for the primary radiation, whereas such a transmission would be further reduced to 37% with a R ration of 16.

In practice, there is a limit for such a ratio of the grids, which is set by the need for an acceptable compromise to be obtained between the anti-scattering effect and the highest possible contrast that can be reached.

A further problem encountered in connection with the utilization of the grids relates to the cancellation of the radiographic image of the same grid. To such a purpose, the grid is installed in a driving mechanism that confers a reciprocating motion to it. It has been found experimentally that there are reciprocating speeds of the grid that bring about stroboscopy effects for displacements of a step or half a step, and that an appropriate selection of the speeds in accordance with the exposure time actually enables such effects to be avoided or at least to be minimized to negligible levels.

US-A-4,982,419, GB-A-2,024,562 and US-A-4,731,806 disclose apparatuses according to the above discussed state of the art.

Conclusively it can however be stated that the overall solution of all of the afore mentioned problems and drawbacks has up to now proved to be practically impossible.

It therefore is a main purpose of the present invention to provide an apparatus for radiological examinations in which the support arrangement for the moving grid is driven in a simple and accurate manner so as to ensure a good radiographic image under the different adjustment and set-up conditions of the apparatus.

According to the present invention, such an aim is reached in the use of means that are adapted to generate a reciprocating motion of the grid at a constant speed, so as this is recited in the appended claims.

The features and advantages of the present invention will be more clearly and readily understandable from the description that is given below by way of non-limiting example with reference to the accompanying drawings, in which:
- Figure 1 is a schematical view of the operating principle of an apparatus for radiological examinations;
- Figure 2 is a schematical view of a detail of the apparatus illustrated in Figure 1;
- Figure 3 is a time-vs-speed diagram showing the curve of displacement through a crank and connecting-rod mechanism of a grid without any speed adjustment provision of the driving motor;
- Figure 4 is a time-vs-speed diagram showing the curve of displacement of a grid with control of the instantaneous speed of the driving motor, according to the present invention;
- Figure 5 is a schematical top view of the apparatus according to the present invention; and

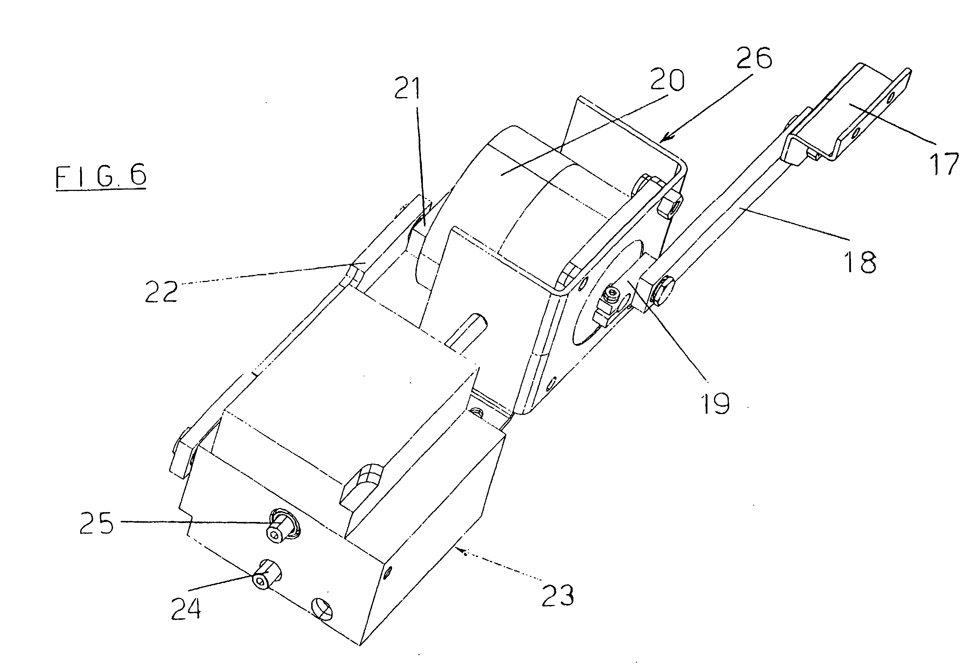

- Figure 6 is a perspective view of a detail of the apparatus illustrated in Figure 5.

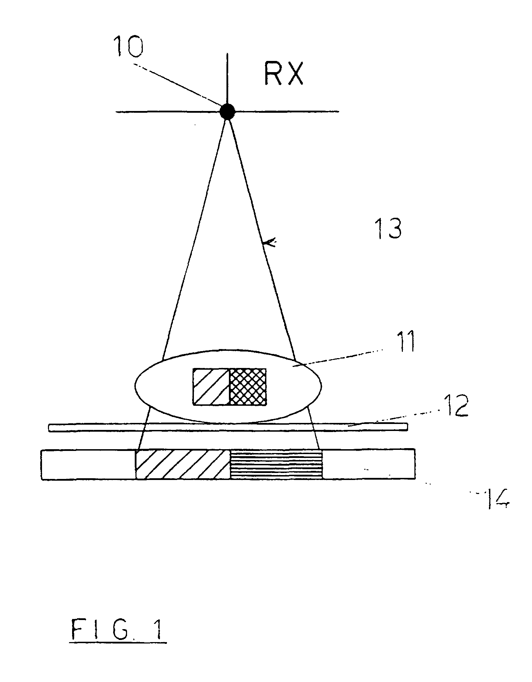

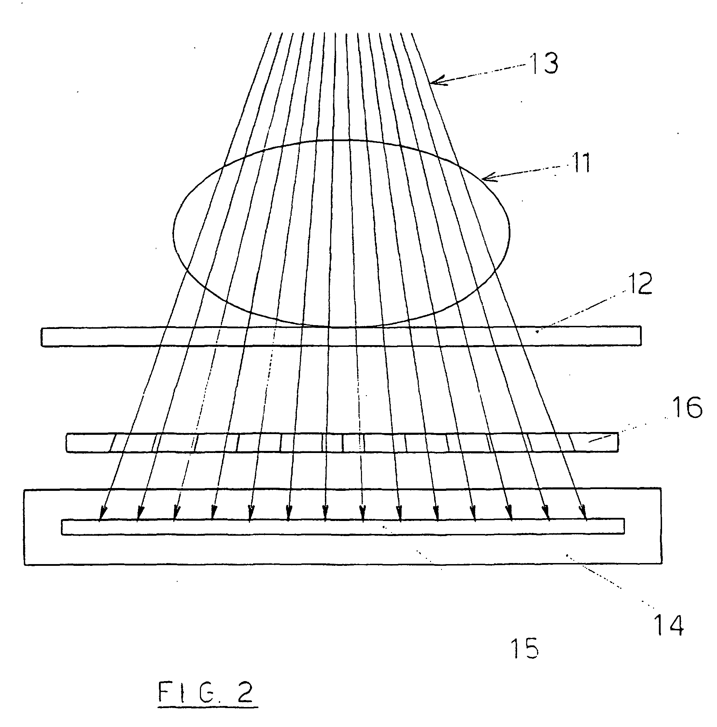

As this is shown schematically in Figures 1 and 2, an apparatus for radiological examinations can be notices to substantially comprise a radiation source (10), in particular a x-ray generator, wherein said radiations are directed towards the body of a patient (11) lying on an appropriate examination table (12). Said radiations (13), after passing through the body of the patient, reach an arrangement (14) which holds the film or plate (15) to be impressed. The arrangement (14) comprises a grid formed by lead strips (16) that are separated from each other by elements of a x-ray transparent material, said grid being displaced according to a reciprocating motion by means of appropriate driving means when the apparatus is operating.

The grid (16) performs the task of filtering the rays scattered by the irradiated elements, since such rays would affect the quality of the image impressed on the photographic film or plate. In order to ensure a good radiographic image, the reciprocating motion of the grid must as far as possible occur at a constant speed.

On the other hand, the grid (16), that is in this way caused to be displaced according to a reciprocating motion, gives rise with its mass to vibrations over the entire structure of the apparatus and, as a result, contributes to a lowered quality of the attainable radiographic image, further to causing a certain inconvenience for the patient who is usually lying quite close to the arrangement (14) receiving the image.

In view of eliminating all such drawbacks, the present invention enables the driving means imparting said reciprocating motion to the grid (16) to be made so as to substantially include a counterweight to balance the oscillating mass of the grid, a stepper motor as the driving means for the grid and the counterweight, a crank-connecting rod mechanism as the motion transmission means from the motor to the counterweight, and a power electronic control arrangement for the stepper motor.

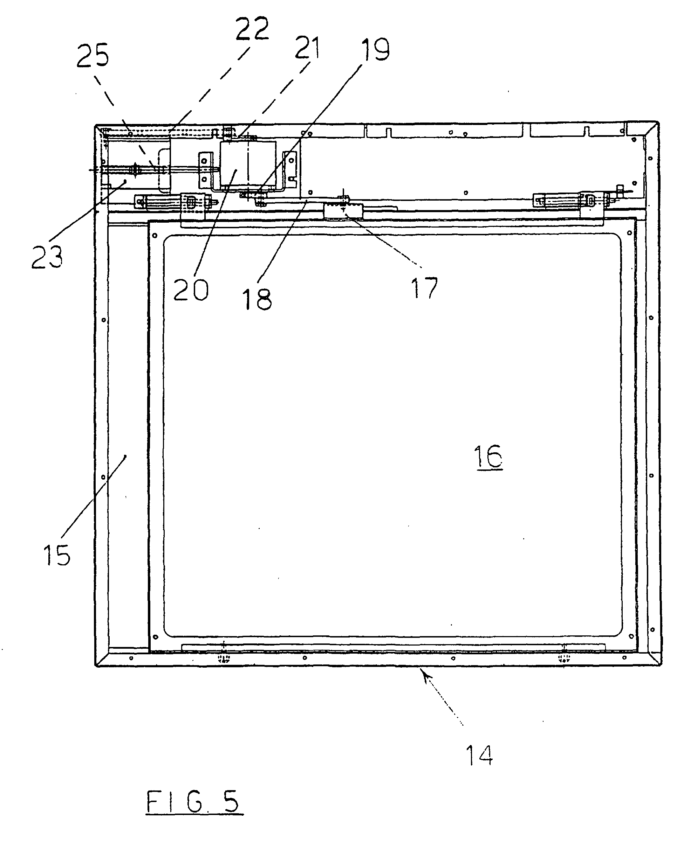

As shown in Figure 5 (in a schematical view from the top) and 6 (in a partially perspective view), the arrangement (14) holding the film or plate (15) to be impressed and the moving grid (16) is housed in a box-like structure on a side of which the various above mentioned means and devices are grouped.

To state it more precisely, the grid (16) is provided with a bracket (17) to which the end portion of a connecting rod (18) is pivotally hinged, the other end portion of said connecting rod being pivotally attached to a crank (19) connected to an end portion of the shaft of a stepper motor (20).

Correspondingly, to the other end portion of the shaft of the motor (20) there is connected a crank (21) to which the end portion of a connecting rod (22) is pivotally attached. The other end portion of said connecting rod (22) is pivotally hinged onto a counterweight (23) that is adapted to balance the mass of the grid (16) in its reciprocating motion. It should be noticed that the cranks (19, 21) of the two motion transmission mechanisms from the motor to the grid and the counterweight, respectively, are in an advantageous manner mounted in a mutually parallel arrangement.

The counterweight (23) is therefore displaced with a rectilinear reciprocating motion in opposition of phase with respect to the grid (16). The counterweight (23) is guided in such a motion thereof by two shafts (24, 25) which are connected with the end portions thereof to the support (26) of the motor (20) and the chassis of the arrangement (14), respectively, and on which said counterweight is able to slide rectilinearly.

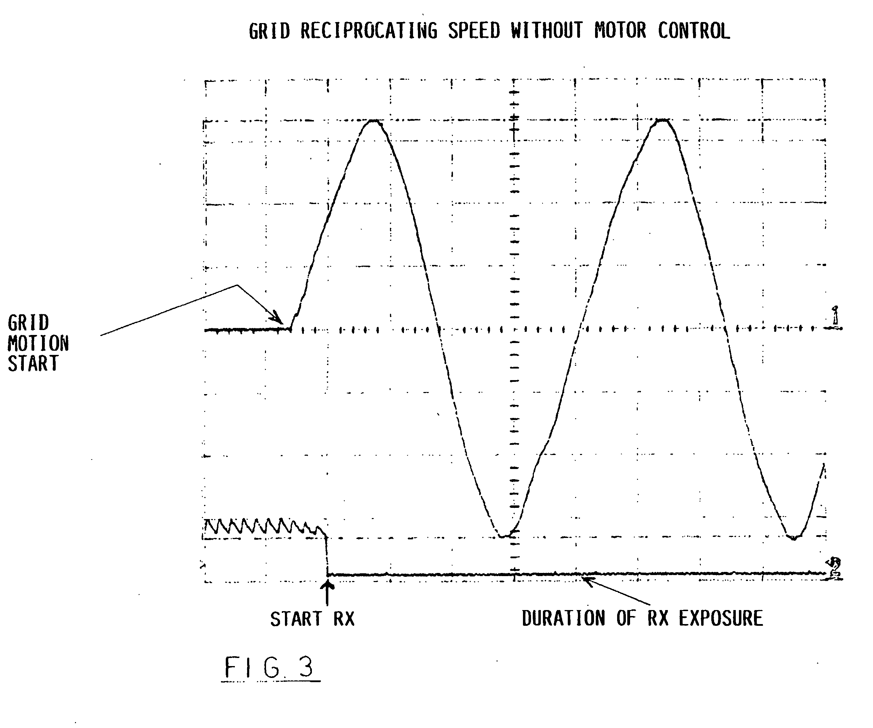

Ideally, the motor (20) turns at a constant speed and the curve of the reciprocating displacement speed of the grid (16), due to the law governing the motion of oscillating masses, should stress a sinusoidal profile (Figure 3). With the apparatus according to the present invention, programmable profiles are on the contrary obtained for the reciprocating speed of the grid (16) under elimination of the imbalances brought about by inertial forces.

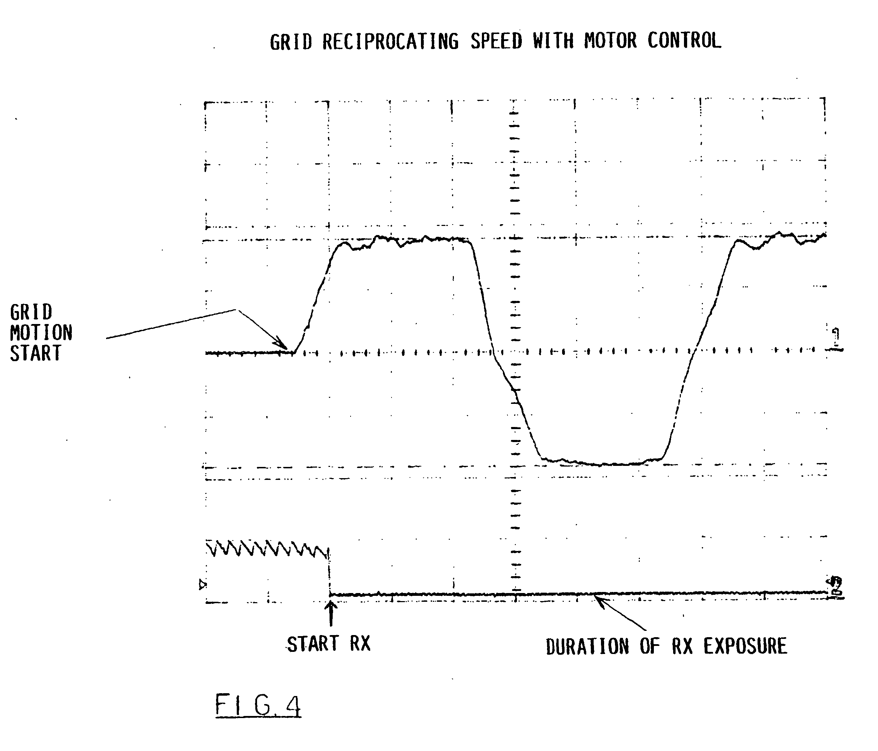

For a profile to be obtained which differs from the sinusoidal one (as shown in Figure 4), the angular speed of the motor needs to be varied on an instant-by-instant basis in accordance with the rotation angle of the crank (19) and the speed profile that should be obtained for the oscillating mass. The variation in the angular speed of the motor is obtained by varying the times elapsing between two successive steps of the motor. Such times are delivered to the power control unit of the motor by the microprocessor-based control arrangement (of a per se known type and, therefore, not shown). As a result, the sinusoidal pattern of the reciprocating speed of the grid (16) brought about by the crank and connecting-rod type of motion transmission system is compensated for through such a motor control.

## Claims

1. Apparatus for radiological examinations comprising a source (10) of radiations that are directed towards the body of a patient and, after passing therethrough, reach an arrangement (14) that holds the film or plate (15) to be impressed, said arrangement comprising a grid (16) formed by an assembly of lead strips, as well as driving means for displacing said grid with a reciprocating motion and a counterweight (23) adapted to balance the oscillating mass of the grid so as to ensure a dynamic balancing of the moving masses, **characterized in that** said driving means of the grid (16) comprise a stepper motor (20) which is provided with an electronic control and is connected to the grid by means of a first motion transmission mechanism of the crank (19) and connecting-rod (18) type.

2. Apparatus for radiological examinations according to claim 1, **characterized in that** also said counterweight (23) is driven by the stepper motor (20) through a second motion transmission mechanism of the crank (21) and connecting-rod (22) type.

3. Apparatus for radiological examinations according to claim 2, **characterized in that** the cranks (19,21) of said first and said second motion transmission mechanisms are both pivotally attached to the shaft of said stepper motor (20) in a mutually parallel arrangement.

4. Apparatus for radiological examinations according to any one of the preceding claims, **characterized in that** the stepper motor (20) is controlled by a power electronic control adapted to vary the angular speed of said motor to the purpose of ensuring the costancy of the reciprocating speed of the grid (16).

## Patentansprüche

1. Vorrichtung für radiologische Untersuchungen, die eine Quelle (10) von Strahlung umfasst, die auf den Körper eines Patienten gerichtet wird und, nachdem sie durch diesen hindurchgetreten ist, zu einer Anordnung (14) gelangt, die den Film oder die Platte (15), der/die zu bedrucken ist, aufnimmt, wobei die Anordnung ein Gitter (16), das aus einer Baugruppe von Leiterstreifen besteht, sowie eine Steuereinrichtung, die das Gitter in einer Hin- und Herbewegung verschiebt, und ein Ausgleichsgewicht (23) umfasst, das die schwingende Masse des Gitters ausgleicht, um ein dynamisches Ausgleichen der sich bewegenden Massen zu gewährleisten, **dadurch gekennzeichnet, dass** die Steuereinrichtung des Gitters (16) einen Schrittmotor (20) umfasst, der mit einer elektronischen Steuerung versehen ist und mit dem Gitter über einen ersten Bewegungsübertragungsmechanismus vom Typ mit Kurbel (19) und Pleuelstange (18) verbunden ist.

2. Vorrichtung für radiologische Untersuchungen nach Anspruch 1, **dadurch gekennzeichnet, dass** auch das Ausgleichsgewicht (23) von dem Schrittmotor (20) über einen zweiten Bewegungsübertragungsmechanismus vom Typ mit Kurbel (21) und Pleuelstange (22) angetrieben wird.

3. Vorrichtung für radiologische Untersuchungen nach Anspruch 2, **dadurch gekennzeichnet, dass** die Kurbeln (19, 21) des ersten und des zweiten Bewegungsübertragungsmechanismus beide in zueinander paralleler Anordnung an der Welle des Schrittmotors (20) angebracht sind.

4. Vorrichtung für radiologische Untersuchungen nach einem der vorangehenden Ansprüche, **dadurch gekennzeichnet, dass** der Schrittmotor (20) von einer Energieelektroniksteuerung gesteuert wird, um die Winkelgeschwindigkeit des Motors zu verändern und so die Konstanz der Geschwindigkeit der Hin- und Herbewegung des Gitters (16) zu gewährleisten.

## Revendications

1. Appareil d'examen radiologique comprenant une source (10) de rayonnements qui sont dirigés vers le corps d'un patient et, après traversée de celui-ci, atteignent un dispositif (14) qui maintient le film ou la plaque (15) devant être impressionnée, ledit dispositif comprenant une grille (16) formée par un assemblage de rubans de plomb, ainsi que des moyens d'entraînement destinés à déplacer ladite grille selon un mouvement de va-et-vient et un contrepoids (23) apte à équilibrer la masse oscillante de la grille afin d'assurer un équilibrage dynamique des masses en mouvement, **caractérisé en ce que** lesdits moyens d'entraînement de la grille (16) comprennent un moteur pas à pas (20) qui est muni d'une commande électronique et est connecté à la grille au moyen d'un premier mécanisme de transmission de mouvement du type à vilebrequin (19) et bielle (18).

2. Appareil d'examen radiologique selon la revendication 1, **caractérisé** également en ce que ledit contrepoids (23) est entraîné par le moteur pas à pas (20) par l'intermédiaire d'un second mécanisme de transmission de mouvement du type à vilebrequin (21) et à bielle (22).

3. Appareil d'examen radiologique selon la revendication 2, **caractérisé en ce que** les vilebrequins (19, 21) desdits premier et second mécanismes de transmission de mouvement sont tous deux fixés de façon pivotante à l'arbre dudit moteur pas à pas (20) selon un agencement mutuellement parallèle.

4. Appareil d'examen radiologique selon l'une quelconque des revendications précédentes, **caractérisé en ce que** le moteur pas à pas (20) est commandé par une commande électronique apte à faire varier la vitesse angulaire dudit moteur dans le but d'assurer la constance de la vitesse de va-et-vient de la grille (16).
